# EUROPEAN PATENT APPLICATION

(11) **EP 3 275 427 A1**
(43) Date of publication of application: **31.01.2018**
(21) Application number: 16182064.2
(22) Date of filing: 29.07.2016
(51) Int. Cl.: A61K 8/37, A61K 8/49, A61Q 9/04, A61K 8/81, A61K 8/92, A61K 8/06

(54) **DEPILATORY COMPOSITION COMPRISING A ROSINOUS TACKIFYING AGENT**

(71) Applicant: Sofibel, 92300 Levallois Perret (FR)
(72) Inventor: SAAID, Amina, 60870 Rieux (FR)
(74) Representative: August & Debouzy avocats

(57) **Abstract**

The invention provides a depilatory composition comprising at least one rosinous tackifying agent, at least one texturizing agent selected from the group consisting of oil, fatty acid ester, wax and mixtures thereof, at least one thermoplastic polymer, at least one surfactant, and water. The invention provides an improved viscosity profile.

## Description

### FIELD OF THE INVENTION

The present invention relates to a depilatory composition, and to its use for removal of unwanted hair from humans.

### TECHNICAL BACKGROUND

Depilatory compositions formed of viscoelastic materials are well known. Known viscoelastic materials include those which are rosin-based. In some products the depilatory compositions may be supplied in the form of strips, retained between cellophane or non-woven sheets. In use, the user peels away one of the sheets, presses the depilatory strip firmly onto the area to be plucked, then pulls one end of the remaining cellophane sheet sharply away from the area.

The hairs trapped in the composition are removed from the treated area along with, optimally, all of the composition, still attached to the remaining backing strip.

In one approach a composition may again be warmed and applied to the skin, for example by means of a spatula or other applicator. The composition is then allowed to set or harden and then pulled sharply to remove the depilatory material and hair from the skin without the necessity of applying a strip of fabric.

It is often a requirement that the composition be first warmed. Warming can be conducted in a variety of ways, for example by placing a container containing the composition in a pan of hot water, into a microwave oven or into an appropriate electrical heating device. A potential problem with such warming methods is that the heating device may not heat the composition consistently throughout and one or more hot regions or hot spots may develop or the product itself may become overheated.

### PRIOR ART

EP1245220 discloses a depilatory composition comprising a rosinous material, water and a surfactant.

WO2005/112876 discloses a depilatory composition comprising a rosinous material, wax, an elastomer, a surfactant and water.

Auchan is presently distributing a product named "cire professionnelle, jambes-maillot-aisselles" which has a composition as indicated on the leaflet as being: glyceryl rosinate, cera alba, paraffin, peg40 hydrogenated castor oil, hydrogenated styrene-butadiene copolymer, aqua, parfum, ethoxydiglycol, glucose, lactic acid, chamomilla recutita flower extract, hexyl cinnamal, caramel, sodium benzoate, linalool, coumarin, potassium sorbate, hydroxyisohexyl 3-cyclohexene carboxaldehyde, alpha-isomethyl ionone, bisabolol, geraniol, limonene, benzvl alcohol, Cl 77019, Cl 77891.

### SUMMARY OF THE INVENTION

The invention provides a depilatory composition comprising at least one rosinous tackifying agent, at least one texturizing agent selected from the group consisting of oil, fatty acid ester, wax and mixtures thereof, at least one thermoplastic polymer, at least one surfactant, and water.

The invention provides the following advantage:
- the composition has an improved viscosity profile, with a low viscosity achieved at a lower temperature, and a more creamy feeling.

According to one embodiment, the depilatory composition comprises, based on the total weight of the depilatory composition:
- from 50 to 90% of at least one rosinous tackifying agent;
- from 7 to 30% of at least one texturizing agent selected from the group consisting of oil, fatty acid ester, wax and mixtures thereof;
- from 1 to 15% of at least one thermoplastic polymer;
- from 1 to 20% of at least one surfactant;
- from 1 to 15% of water.

According to one embodiment, the depilatory composition comprises, based on the total weight of the depilatory composition:
- from 60 to 80% of at least one rosinous tackifying agent;
- from 15 to 20% of at least one texturizing agent selected from the group consisting of oil, fatty acid ester, wax and mixtures thereof;
- from 2 to 6% of at least one thermoplastic polymer;
- from 2 to 8% of at least one surfactant;
- from 2 to 6% of water.

According to one embodiment, the rosinous tackifying agent comprises glyceryl rosinate and methyl hydrogenated rosinate.

According to one embodiment, the texturizing agent is a wax. The wax can be selected from the group consisting of paraffin, beeswax, microcrystalline wax and mixtures of two or more thereof, preferably a mixture of paraffin and microcrystalline wax.

According to one embodiment, the thermoplastic polymer is polyethylene.

According to one embodiment, the surfactant is polysorbate, preferably polysorbate 80.

According to one embodiment, the depilatory composition comprises, based on the total weight of the depilatory composition:
- from 60 to 80% of glyceryl rosinate and methyl hydrogenated rosinate;
- from 15 to 20% of paraffin and microcrystalline wax;
- from 2 to 6% of polyethylene;
- from 2 to 8% of polysorbate;
- from 2 to 6% of water.

The invention also provides for a microwaveable container comprising the depilatory composition according to the invention.

The invention also provides for a method for removing hair from skin which comprises:
(a) heating a depilatory composition according to the invention;
(b) applying said composition to the skin;
(c) allowing said composition to set and hold said hair or applying a strip of material over said composition and allowing said strip to adhere thereto; and;
(d) removing said composition and hair by peeling said composition or said material and said composition, from the skin.

According to one embodiment, said composition is heated by microwaves.

The invention further provides advantages as will be apparent from the description.

### DESCRIPTION OF EMBODIMENTS OF THE INVENTION

The invention is now disclosed in more details in the following description. Unless specified otherwise, amounts and percentages are by weight.

### Rosinous tackifying agent.

The tackifying agent is a rosinous material, typically glyceryl rosinate, alone or in combination with triethylene glycol rosinate or methyl hydrogenated rosinate, the latter being preferred. Triethylene glycol rosinate usually represents the majority of the rosinous material when used in the referenced combination, typically about 5 to 20, preferably 7 to 10 times more than the partner component.

The overall amount of the resinous material is typically from 50% to 90%, preferably from 60% to 80% of the total weight of the depilatory composition.

### Texturizing agent

A texturizing agent can be added to the tackifying component, the rosinous material. The texturizing agent can be an oil, an ester of a fatty acid, or a wax, the latter being described in more detail below. The oil can be a natural or synthetic oil, such as a paraffinic oil. The ester may be a known ester of fatty acid, where the acid can comprise from 12 to 30 carbon atoms, preferably from 14 to 20.

### Wax.

As stated above, wax may be present in the composition as a texturizing agent (and also as a viscosity adjuster). Suitable waxes are natural waxes such as beeswax, lanolin and hydrocarbon waxes such as microcrystalline wax or paraffin wax. Desirably, a mixture of two waxes is preferred, and can be a mixture of at least one natural wax and at least one synthetic wax, or a mixture of at least two synthetic or a mixture of at least two natural waxes. A preferred mixture is a mixture of paraffin wax and microcrystalline wax, according to varying ratios, e.g. from 5:1 to 1:5, preferably 1:2 to 3:1.

The overall amount of texturizing agent is typically from 7% to 30%, preferably from 15% to 20% of the total weight of the depilatory composition.

### Polymer.

A polymer is also present which is a thermoplastic polymer.

A thermoplastic polymer is typically a polyolefin such as polyethylene (PE). The PE can have varying hardness (e.g. values at 25°C in dmm from 0.2 to 8), varying drop point Mettler (e.g. from 90 to 130°C), varying viscosities (brookfield at 140°C in cps, e.g. from 100 to 1000).

The nature of the polymer, which is typically not elastomeric, allows notably an improved viscosity profile compared to the prior art as represented by WO2005/112876. Notably, the composition of the invention, compared to the prior art, has a viscosity profile such that the viscosity of the composition of the invention reaches a value of 100 Pa.s at a temperature that is lower than the prior art. At such lower temperatures, the viscosity of prior art compositions is significantly higher than the viscosity of the composition of the claimed invention. Also, the viscosity profile is such that the viscosity of the composition of the claimed invention will gently decrease and remain at acceptable values even at higher temperatures, compared to the prior art which provides for compositions which reach a viscosity substantially zero at temperatures not even 10°C higher than the temperature at which their viscosity is at 100 Pa.s. The viscosity is a plane-plane viscosity, with the following settings:
- apparatus: Rheometer Discovery DHR-1 (TA Instruments)
- operating conditions: oscillating mode
- plane-plane gap 800 microns
- sample is deposited at 100°C with a spatula
- sample is conditioned at 100°C with a preshear of 2 minutes at 10s⁻¹
- temperature ramp from 100°C to 10°C at 3°C/min
- frequency 10s⁻¹

The softening point (as measured using the ring-and-ball method) of the compositions of the invention is also 10 to 15°C higher than the softening point of the prior art compositions. The overall result is also a feeling for the user of a more creamy texture for the composition of the invention, which is more pleasant for the user.

The overall amount of the thermoplastic polymer is typically from 1% to 15%, preferably from 2% to 6%.

### Surfactant

The surfactant may be, for example, a nonionic, anionic, cationic, amphoteric or zwitterionic surfactant or mixture thereof. Examples of nonionic surfactants are alkoxylated fatty alcohols such as fatty alcohols containing from 8 to 18 carbon atoms, especially from 12 to 16 carbon atoms, with from 2 to 10 ethoxy groups, especially 3 to 7 ethoxy groups. Further examples of surfactants are alcohol-oil transesterified surfactants such as PEG hydrogenated oils, sorbitan fatty acid esters, polyethylene glycol sorbitan fatty acid esters, sterol and sterol derivatives.

Suitable surfactants are especially polyethylene glycol sorbitan fatty acid esters, also known as polysorbate. A preferred one is polysorbate 80.

The overall amount of surfactant is typically from 1% to 20%, preferably from 2% to 8% of the total weight of the depilatory composition.

### Water

Water is also present in the composition. Water allows a better temperature profile, including a lower application temperature.

The overall amount of water is typically from 1% to 15%, preferably from 2% to 6% of the total weight of the depilatory composition.

### Other ingredients

Any other ingredient commonly used can be present. For example, perfumes and essential oils can be incorporated if it is so wished. Preservatives, coloring agents and the like can also be present, albeit this is generally not necessary.

### Method of making.

The compositions of the present invention may be prepared by a variety of methods. For example, the rosinous material may be melted together with the wax and the polymer. The final temperature should preferably not exceed 95°C. Separately water may be mixed with the surfactant, and then added to the molten rosinous material and mixed in until the composition is homogeneous. Both phases can have the same or a similar temperature when they are mixed, or the water portion may be at room temperature or some other temperature when it is added to the rosinous portion. The mixture may then be cooled down to room temperature. Other ingredients may be added either to the rosinous portion or the water portion, depending on their properties and solubilities.

### Container.

The container may be of a traditional design such as is presently found in the art.

### Method of using.

When used, the composition of the present invention may simply be heated. Heating may be carried out by any means such as by placing a container comprising the composition in a bath of hot water, such as a saucepan of boiling water. Most desirably, however, the composition of the present invention can be heated in an oven or a microwave oven. The length of time that the composition must be heated will depend on a number of factors, including the quantity of composition to be heated, the nature of the container in which it is held and the power of the oven. Heating times in a microwave oven at maximum power (e.g. 900W or 1000W) can range from 45 seconds to 3 minutes, depending on the quantity being heated. Suitable heating instructions may be provided in association with the container containing the composition. Once the composition has been warmed, it may be stirred, if desired, in particular to ensure a more uniform temperature throughout the composition.

The depilatory may then be applied by a user to the skin, for example using a spatula or applicator. The optimal application temperature is preferably about 45-55°C. The composition is then allowed to set on the skin and hold the hair, or a strip of material such as a fabric can be applied over the composition and allowed to adhere thereto. The composition, or the composition and the fabric together, but preferably the composition without any fabric, can then be pulled or peeled from the skin in order to remove the hair therefrom. There is no minimum length for the hair in order for the hair to be removed satisfactorily.

The composition exhibits a creamy texture at typical use temperatures such as those described herein..

The compositions of the present invention are such that residues are removable from the user's skin by washing with an oily material such as (paraffin) oil.

### EXAMPLES

The following further examples illustrate the invention without limiting it.

### Example 1

The following composition for a 400g weight was prepared.

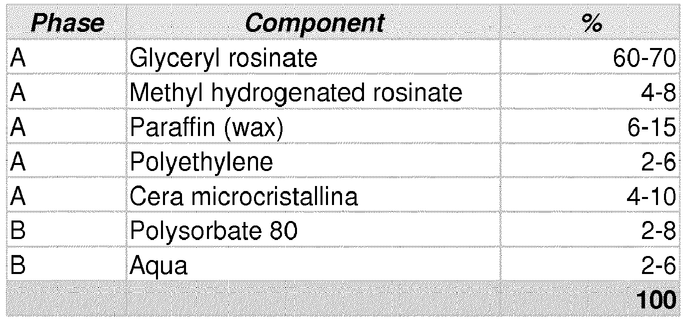

### Example 2

The composition of example 1 is useful for hair removal.

It was applied at a temperature of 50-55°C, and provided stiff strips once solidified on the user's skin.

The composition was heated in a microwave oven. The composition was placed in a 1000W oven for a duration of 1 minute and 15 seconds. The temperature was measured at one point in the center of the composition, half way down the pot at a value of about 54°C, with a low heterogeneity.

No rest time was needed before applying the composition of the invention.

The oxidative induction time for the composition of example 1 was similar to existing compositions on the market. Further the viscosity profile of the composition of example 1 was such that:
- the softening point was about 70-75°C
- the viscosity at 50°C was lower than 100 Pa.s

A clinical study was performed in order to evaluate the hair removal efficacy, the cutaneous tolerance, organoleptic characteristics.

The assessment criteria were hair removal efficacy (quantitatively counting of hair numbers on skin before and after depilation), subjective evaluation (questionnaire), cutaneous tolerance (clinical examination and collection of unpleasant sensations). The reference product was the product Monoi® of the applicant.

42 patients were enrolled for waxing on the body, 23 for waxing on the face. The average age was 36±2 years (between 18 and 54).

The main inclusion criteria were:
- Sex: female.
- Age between 18 and 55
- Subject being used to waxing the zones of the study.
- Subject with numerous hair on the zones of the study.
- Subject having removed hair by the same method two weeks before enrollment for 2mm of hair length.
- Subject with sensitive skin on the face and the body (50%).
- Subject with other type of skin on the face and the body (50%).

Under these study conditions, the composition of the invention is as effective on the hair removal as the reference Monoi®, with no significant difference concerning the counting of hairs on the armpits, half-legs, eyebrows and upper lip, is better-tolerated than reference product on the body, and is appreciated by the subjects and by the beauticians for its characteristics and for its efficacy, for its convenient temperature of application, its creamy texture and its ease of homogenization.

## Claims

1. Depilatory composition comprising:
- at least one rosinous tackifying agent;
- at least one texturizing agent selected from the group consisting of oil, fatty acid ester, wax and mixtures thereof;
- at least one thermoplastic polymer;
- at least one surfactant; and
- water.

2. Depilatory composition of claim 1 comprising, based on the total weight of the depilatory composition:
- from 50 to 90% of at least one rosinous tackifying agent;
- from 7 to 30% of at least one texturizing agent selected from the group consisting of oil, fatty acid ester, wax and mixtures thereof;
- from 1 to 15% of at least one thermoplastic polymer;
- from 1 to 20% of at least one surfactant;
- from 1 to 15% of water.

3. Depilatory composition of claim 1 or 2 comprising, based on the total weight of the depilatory composition:
- from 60 to 80% of at least one rosinous tackifying agent;
- from 15 to 20% of at least one texturizing agent selected from the group consisting of oil, fatty acid ester, wax and mixtures thereof;
- from 2 to 6% of at least one thermoplastic polymer;
- from 2 to 8% of at least one surfactant;
- from 2 to 6% of water.

4. Depilatory composition of any one of claims 1 to 3, wherein the rosinous tackifying agent comprises glyceryl rosinate and methyl hydrogenated rosinate.

5. Depilatory composition of any one of claims 1 to 4, wherein the texturizing agent is a wax.

6. Depilatory composition of claim 5, wherein the wax is selected from the group consisting of paraffin, beeswax, microcrystalline wax and mixtures of two or more thereof, preferably a mixture of paraffin and microcrystalline wax.

7. Depilatory composition of any one of claims 1 to 6 wherein the thermoplastic polymer is polyethylene.

8. Depilatory composition of any one of claims 1 to 7, wherein the surfactant is polysorbate, preferably polysorbate 80.

9. Depilatory composition of any one of claims 1 to 8 comprising, based on the total weight of the depilatory composition:
- from 60 to 80% of glyceryl rosinate and methyl hydrogenated rosinate;
- from 15 to 20% of paraffin and microcrystalline wax;
- from 2 to 6% of polyethylene;
- from 2 to 8% of polysorbate;
- from 2 to 6% of water.

10. A microwaveable container comprising the depilatory composition of any one of claims 1 to 9.

11. A method for removing hair from skin which comprises:
(a) heating a composition as defined in any one of the previous claims;
(b) applying said composition to the skin;
(c) allowing said composition to set and hold said hair or applying a strip of material over said composition and allowing said strip to adhere thereto; and;
(d) removing said composition and hair by peeling said composition or said material and said composition, from the skin.

12. The method of claim 11 said composition is heated by microwaves.
